# EUROPEAN PATENT SPECIFICATION

(11) **EP 0 928 831 B1**
(45) Date of publication and mention of the grant of the patent: **03.05.2006**
(21) Application number: 97309957.5
(22) Date of filing: 10.12.1997
(51) Int. Cl.: C12N 15/31, C12N 15/01, C07K 14/28, A61K 39/106, C12R 1/63

(54) **Cholera organisms, vaccines and preparation thereof**
Choleraorganismen, Impfstoffe und ihre Verwendung
Organismes cholerae, vaccins et leur préparation

(43) Date of publication of application: 14.07.1999
(73) Proprietor: COUNCIL OF SCIENTIFIC AND INDUSTRIAL RESEARCH, New Delhi 110 001 (IN); National Institute of Cholera and Enteric Diseases, Calcutta 700 010 (IN); DEPARTMENT OF BIOTECHNOLOGY, MINISTERY OF SCIENCE AND TECHNOLOGY, GOVERNMENT OF INDIA, New Delhi 110003 (IN)
(72) Inventor: Muthukumarappa, Thungapathra, Union Territory of Chandigarh 160036 (IN); Ghosh, Amit, Union Territory of Chandigarh 160036 (IN); Sharma, Charu, Union Territory of Chandigarh 160036 (IN); Gupta, Naveen, Union Territory of Chandigarh 160036 (IN); Ghosh, Ranajit K. Indian Inst. of Chem. Biology, Jadavpur Calcutta 700 032 (IN); Mukhopadhyay, A. Nat.Inst.of Cholera/Enteric Dis., Calcutta 700 010 (IN); Kole, Hemanta Nat.Inst.of Cholera/Enteric Diseases, Calcutta 700 010 (IN); Nair,G.B. Nat.Inst.of Cholera/Enteric Diseases, Calcutta 700 010 (IN)
(74) Representative: Ablewhite, Alan James

(56) References cited:
- EP-A- 0 125 228
- EP-A- 0 581 329
- WO-A-91/18979
- WO-A-94/01533
- WO-A-95/10300
- WO-A-95/18633
- J. MICHALSKI ET AL.: "CDV110, an attenuated Vibrio cholerae O1 El Tor live oral vaccine strain" INFECTION AND IMMUNITY, vol. 61, no. 10, October 1993, AM. SOC. MICROBIOL.,, pages 4462-4468, XP002065119

## Description

### FIELD OF THE INVENTION

The present invention relates to a process for the isolation of a *V. cholera* strain and its use in the preparation of cholera vaccine. The present invention also, particularly, relates to a process for the preparation of cholera vaccine using *V*. *cholerae* strain deposited at Institute of Microbial Technology, Chandigarh, India, one of the constituent laboratories of the applicants, having Accession No. B0010 which is also deposited at American Type Culture Collection, Rockville, Maryland, USA and having Accession No.ATCC 202010. The vaccine prepared by the process of the invention has proved efficacious in animal trials. If proved successful in human trials, it can used to control the disease cholera, since an effective and safe cholera vaccine is still not available. The present invention specifically relates to a process for the preparation of cholera vaccine using V. *cholerae* strain having the Accession No. B00.10 (ATCC No.202010) as a parent strain.

**The various scientific terms used in this specification are described in alphabetical order as given below :**

Annealing refers to the process in which single strands of deoxyribonucleic acid (DNA) having complementary base sequences become paired to form a double stranded molecule.

Clone refers to a large number of cells or plasmid molecules derived from a single ancestral cell or plasmid molecule and a colony refers to a visible cluster of cells formed on a solid growth medium by repeated division of a single parental cell.

Cloning in recombinant DNA technology refers to the linking of a specific gene or DNA fragment to a replicable DNA molecule such as a plasmid or phage DNA.

Colony refers to a visible cluster of cells formed on a solid growth medium by repeated division of a single parental cell.

Colonization refers to the ability of a bacterium to remain at a particular site and multiply.

Denaturation refers to conversion of DNA from double stranded molecule into the single stranded form.

DNA refers to the macromolecule, deoxyribonucleic acid formed from covalently linked deoxynucleotide units.

DNA ligase is an enzyme that joins two DNA molecules through phosphodiester bond.

DNA polymerase is an enzyme that catalyses the synthesis of DNA from deoxynucleotides under the direction of a DNA template strand.

DNA sequencing refers to determination of the order of nucleotides in a DNA molecule.

Electrophoresis is a technique used to separate molecules on the basis of their different migration rates (due to their molecular size difference) in a solid support in response to an applied electric field.

Electroporation refers to the introduction of DNA molecules into a cell by means of an electric field.

Enterotoxin refers to a protein toxin secreted by bacteria that acts specifically on the intestinal mucosa.

Gene refers to the hereditary unit containing genetic information that is transcribed into ribonucleic acid (RNA) which is processed and either functions directly or is translated into a polypeptide chain. Genome refers to the total genetic information carried by a cell. Homologous recombination refers to genetic exchange between identical DNA sequences.

Hybridization refers to the process whereby two complementary nucleic acid strands form a double helix during an annealing period; a powerful technique for detecting specific nucleotide sequences.

Immunity refers to the resistance of an organism to disease causing agents.

Immunogen is an antigen that induces an immune response.

Oligonucleotide refers to a short single stranded nucleic acid.

Operon refers to a group of contiguous genes that are transcribed into a single messenger ribonucleic acid (mRNA) molecule from a single promoter.

Plasmid is an extra chromosomal genetic element that replicates independant of the host chromosome; used as cloning vector.

Polymerase chain reaction is a technique for amplifying specific regions of DNA by multiple cycles of polymerisation, each followed by a brief heat treatment to separate complementary strands.

Primer refers to an oligonucleotide that can hybridize with a longer DNA strand and can be extended by DNA polymerase for example in polymerase chain reaction.

Probe refers to a radioactive DNA molecule used in DNA-DNA hybridization assay.

Promoter refers to a specific DNA sequence at which RNA polymerase binds and initiates transcription.

Protein refers to the linear polymer of amino acids linked together by peptide bonds in a specific sequence.

Recombinant DNA technology refers to procedures for creating new DNA molecule by joining DNA segments from different DNA molecules.

Recombination is a process in which chromosomes or DNA molecules are broken and then rejoined in new combination.

Restriction enzyme is a nuclease that recognizes a short nucleotide sequence (restriction site) in a DNA molecule and cleaves the molecule at that site.

Ribosome binding site or shine Dalgarno sequence is the base sequence in a prokaryotic mRNA molecule to which a ribosome binds to initiate protein synthesis.

Selection refers to a procedure designed in such a way that only a desired type of cell can survive as in selection for resistance to an antibiotic.

Southern hybridization is a process in which following electrophoretic separation of nucleic acids, denatured DNA is transferred from gel to a membrane filter and then exposed to radioactive DNA probe under conditions of renaturation. The radioactive regions locate- the DNA segments homologous to the probe.

Start codon refers to the triplet sequence AUG on the mRNA molecule from which translation into the polypeptide chain starts.

Sticky end refers to a single stranded region at the end of double stranded DNA molecule that is complementary to a single stranded region at the other end of the same molecule or at the end of a different molecule.

Stop codon refers to one of the three mRNA codons UGG, UAA, UGA at which polypeptide synthesis stops.

Subunit refers to a polypeptide chain that is part of a protein containing several polypeptide chains.

Suicide vector refers to the plasmid vector which cannot replicate in a host cell that does not provide necessary component for its replication.

Tandem duplication refers to repetition of a DNA segment on a chromosome in contiguous array in the same orientation.

Template refers to a nucleic acid strand whose base sequence is copied in a polymerase chain reaction.

Transcription refers to the process by which the information contained in the coding strand of DNA is copied into a single stranded DNA molecule of complementary base sequence.

Transcription activator is a positive control element that stimulates transcription by binding to particular sites in DNA.

Transcription terminator is a sequence of DNA, represented at the end of RNA transcript that causes RNA polymerase to terminate transcription.

Transcription start site is the base from which the DNA is transcribed to RNA molecule.

Transformation refers to introduction of DNA into a bacterial cell.

Translation refers to the process by which the amino acid sequence of a polypeptide chain is derived from the nucleotide sequence of a mRNA molecule associated with a ribosome.

Vector refers to a plasmid cloning vehicle through which a DNA segment can be carried from one organism to another.

Cholera is a lethal diarrhoeal disease caused by the gram negative bacterium *Vibrio cholerae* (*V. cholerae*). This disease which has killed millions of people continues to be a major health hazard worldwide affecting about half-a-million people every year. Today there is hardly any country in the world which is not affected by it and according to World Health Organisation (WHO), even Europe, which had been reporting only imported cases of cholera, registered a 30 fold increase in the indigeneous cholera cases in 1994.

The severe diarrhoea that occurs in cholera is the result of host reaction to an extracellular enterotoxin called cholera toxin. The cholera toxin consists of two different protein subunits : encoded by the genes *ctx*A and *ctx*B which form a single operon called ctx AB (or the ctx operon). A single A subunit and five B subunits make up the complete toxin molecule. It is the A subunit of the cholera toxin which is responsible for the fluid loss by upsetting the fine control of water and electrolyte balance of the intestinal epithelial cells. The B subunits bind to the host intestinal membrane and perhaps aid the entry of the catalytic A subunit into the host mucosal cells. The B subunit is also immunogenic and is capable of eliciting antitoxic immunity in the host.

### PRIOR ART REFERENCES

In order to control/prevent cholera, various vaccines have been developed. Whole cell killed vaccine administered parenterally is still being used in developing countries but they offer only about 50% protection, that too for several months only. [(i) Fellay, J.C. and Gangarosa (1978) in cholera and related diarrheas (Ouchterlony, O. & Holmgren J. eds.) 43rd Nobel Symp. Stockholm pp 204-210, Karger, Basel. (ii) Svennerholm, A.M., Helmgren, J., Hanson, L.A., Lindblad, B.A. Quereshi, F. and Rahimtoola, R.J. (1977) Scand J Immunol 6, 1345-1349. (iii) Svennerholm, A.M., Hanson, L.A., Holmgren J., Lindblad, B.S., Nilsson, and Quereshi, F. (1980) Infect Immun 30, 427-430]. After the advent of recombinant DNA technology, attenuated live oral *Vibrio cholerae* strains lacking cholera toxin A subunit turned out to be attractive candidates since they would mimic infection derived immunity by colonizing the intestine and stimulate both antibacterial and antitoxic immunity. [(i) Kaper, J.B., Lockman, H., Baldini, M.M. and Levine, M.M. (1984) Nature 308 655-658. (ii) Kaper, J.B., Lockman, H., Baldini, M.M. and Levine, M.M. (1984) Biotechnology 2, 345-349. (iii) Mekalanos, J.J., Swartz, S.J., Pearson, G.D.N., Harford, N., Groyne, F. and M. de Wilde (1983) Nature 306, 551-557]. But all such attenuated *Vibrio cholerae* O1 vaccine strains developed so far were still found to cause mild to moderate diarrhoea when tested on volunteers. [(i) Levine, M.M., Kaper, J.B., Herrington, D.A., Losonsky, G., Morris, J.G., Clements, M.L., Black, R.E., Tall, B. and Hall, R. (1988) Infect Immun 56, 161-167. (ii) Herrington, D., Hall, R., Losonsky, G., Mekalanos, J.J., Taylor, R.K. and Levine, M.M. (1988) J Exp Med 168, 1487-1492]. It was subsequently discovered that virulent strains of *V. cholerae* possess genes for other toxins besides the cholera toxin. It was further discovered that ctxAB genes are actually a part of a virulence cassette which encodes four other virulence genes namely zot, ace, cep and orfU besides the ctx. The genes encoding all these factors reside in a mobile genetic element. Within this element all the above genes are present as a contiguous array in what is known as core element. The core element is flanked on both sides by a 2.7 kb repetitive sequence called RS1 element. The RS1 element encodes a site specific recombination system which is responsible for the recombinase A independent integration, duplication and amplification of the toxin cassette. Attempts were therefore made next to create a vaccine strain in which all these known virulence genes *ctx*A, zot, ace etc. were deleted. [Michalski, J., Galen, J.E., Fasano, A., and Kaper, J.B. (1993) Infect Immun 61, 4462-4468]. However, when such a strain was tested it was found to be equally reactogenic as other vaccine prototypes which were not devoid of *zot, ace* etc. [Tacket, C.O., Losonsky, G., Nataro, J.P., Cryz, S.J., Edelman, R., Fasano, A., Michalski, J., Kaper, J.B. and Levine, M.M. (1993) J Infect Dis 168 1536-1540].

Recently, the applicants have come across two South African Patent Nos.93/4736 and 95/0082 relating to cholera vaccine. However,the starting strains described in the South African Patent are :
1. Peru-2
2. Bang-2
3. Bah-2
4. Bengal-2

Nos. 1-3 are described in Taylor et al (1994) J. Infect Diseases 170, 1518-23. These are NOT Natural isolates but are genetically engineered strains to produce ctx. South African patents describe development of Soft Agar Penetration defective mutants from these.

No.4 is described in Waldor & mekalanos (1994) J. Infect.Diseases, 170, 278, Bengal-2 Lis genetically engineered construct and NOT a natural ctx from V. Cholera. M010 is a V. cholerae 0139 strain. South African Patents describe development of Soft Agar Penetration defective mutant from this.

On the other hand, starting strain of the present invention is a naturally occuring strain. This was engineered to create the vaccine strain which has been deposited at American Type Culture Collection, 12301 Parklawn Drive, Rockville,Maryland 20852 USA and has been assigned the Accession No. 202011 which produces only the immunogenic "B" subunit of the cholera toxin.

Inspite of the good protection offered by the various vaccine candidates developed by such "deletional" approach their main drawback was that all of them were capable of causing mild to moderate diarrhoea. The reactogenicity of these earlier vaccine candidates was probably dependent to a large extent on unknown factors present in the parent strains. To circumvent this problem in developing an ideal cholera vaccine which is safe (i.e. non-reactogenic) and highly protective, an alternative approach would be to develop a vaccine from a strain which is non-toxinogenic in biological assays and is devoid of all known virulence genes.

### OBJECTS OF THE INVENTION

Thus, the main objective of the present invention is to provide a process for the preparation of cholera vaccine.

Another objective of the present invention is to provide a process for the preparation of cholera vaccine using a strain of *Vibrio cholerae* having Accession No. B0010 (ATCC . 202010) .

Yet another object of the invention relates to producing a cholera vaccine having Accession No. B0011 (ATCC 202011) which has been constructed by incorporating the immunogenic ctxB subunit gene into the chromosome of the V. cholerae strain having Accession No.B0010 (ATCC 202010).

### SUMMARY OF THE INVENTION

Accordingly, the present invention provides a method of producing a recombinant V. cholerae strain as defined in Claim 1, and a vaccine comprising said strain. A process for the preparation of a cholera vaccine useful for preventing cholera comprises :
a. isolating *V. cholerae* from the stool of a patient suffering from cholera by spreading the stool on a selector medium specific for *V. cholerae*
b. Separating a *V. cholerae* strain from the population of the strains isolated, the strain having been deposited at Microbial Type Culture Collection (MTCC) at the Institute of Microbial Technology (IMT), Chandigarh, a constituent laboratory of the applicants and having the accession no. B0010 (ATCC 202010), and
c. Incorporating immunogenic cholera toxin (ctx) B subunit gene into the chromosome of the strain having the accession no. MTCC B0010 (ATCC 202010) by conventional method to produce the vaccine.

### DETAILED DESCRIPTION OF THE INVENTION

The main finding of the present invention is the integration of the immunogenic B Subunit gene of the cholera toxin into the chromosome of non-toxinogenic strain of V. *cholerae* by targeted genetic recombination, at the hemolysinA gene (hlyA) resulting in the disruption of hlyA.

The present invention is derived from a strain of *V. cholerae* of 01 serotype which is devoid of known virulence genes and is a good colonizer. The strain is a clinical isolate recovered from a patient with cholera and is identified as *Vibrio cholerae* of the O1 serogroup, Inaba serotype and belonged to the Eltor biotype. This strain is selected after screening several hundred strains of *V. cholerae* using a battery of DNA probes specific for virulence associated factors including cholera toxin, zonula occludens toxin, accessory cholera enterotoxin. This strain does not possess the virulence package normally located on a 4.5 kb region in toxinogenic strains and therefore does not produce cholera toxin or the other known secondary toxins which reportedly exacerbate the fluid accumulation. This strain, however, has the E1 Tor hemolysin A gene, which would be a suitable target on the chromosome for the integration of the immunogenic ctxB subunit gene to make it a producer of the B subunit of cholera toxin. This strain also has the toxin coregulated pilus A (tcpA) gene which is required for colonization of the strain in the intestine, and the toxR gene which is required for promoting the expression of the ctxB subunit gene. The reasons for selecting the strain having the MTCC accession no. B0010 as a starting strain for developing an oral recombinant vaccine are as follows:
1. The strain having accession no. B0010 (ATCC 202010) is not capable of accumulating fluid in the ligated ileal loop rabbit model indicating that the strain does not produce any secretogenic factors.
2. The strain does not produce any adverse effect on rabbits when examined by the RITARD model; in other words the strain is not reactogenic .
3. Despite non-reactogenicity, this strain displays impressive colonization ability in the rabbit model and this is probably due to the presence of the toxin coregulated pilus whose presence was determined directly by Southern hybridization studies, an important factor assisting colonization of *Vibrio cholerae.* It has been found that when the ctxB gene is introduced with its own promoter and its own Shine Dalgarno sequence into the chromosome of this strain, it ensures optimum expression of the gene in the gut. It has also been found that the recombinant *V. cholerae* clones thus created show up as an efficacious vaccine in animal model studies.

### The detailed procedure of this invention are given below :

### Isolation of the parent V. cholerae strain having MTCC accession no. B0010 (ATCC 202010)

Stool specimens from cholera patients were collected in sterile MacCartney bottles with sterile catheters. Soon after collection stool specimens were transported to the laboratory and examined within 2 hours for *V. cholerae* and for other common toxinogenic enteropathogens such as enterotoxinogenic *E. coli* (ETEC), Shigella, Salmonella and Campylobacter spp. by standard published techniques (W.H.O. Program for control of diarrhoeal diseases (CCD/83.3 Rev.1), Manual for laboratory investigations of acute enteric infections, Geneva, W.H.O., 1987).

*V. cholerae* strains from the stool specimens were isolated by plating the stool specimens on a selective medium like thiosulfate-citrate bile salts sucrose agar (TCBS), tellurite taurocholate gelatin agar (TTGA), Vibrio agar, sucrose tellurite teepol medium and polymyxin mannose tellurite agar which are specific for *V. cholerae.* The *V. cholerae* strains that had grown as colonies were collected manually. Several hundred strains so obtained were grown in media specific for identification of V. *cholerae* by biochemical tests. The several hundred strains that were confirmed to be *V. cholerae* 01 (of serotype) were grown as individual colonies on Luria broth agar for screening by hybdridization with DNA probes specific for virulence genes ctx, zot and ace. The colony hybridization experiments were performed by known methods. The strains that were found to be devoid of the virulence genes viz ctx, zot, ace were tested for cholera toxin like and other cytotoxin activities by known methods (Oku Y, Uesaka Y, Hirayama T, Takeda Y. Microbial Immunol 1988 : 32 : 807-816 and Nair G.B., Oku Y, Takeda Y. et al. Appl. Environ. Microbiol 1988; 54 : 3180-3182).

One of the strains did not have the virulence genes and did not have cholera toxin like and other cytotoxin activities. This *V. cholerae* is the strain having the Accession No. B0010 (ATCC 202010). In this strain the presence of the cryptic hemolysin A gene was ascertained by hybridization with hlyA probe. This is essential since the integration of ctxB subunit gene for the preparation of cholera vaccine has to be carried out by targeted recombination at hlyA locus. Since the product of tcpA gene is the main component of the pilus required for effective colonization of *Vibrio cholerae* in the intestine, its presence was also ascertained by hybridization with tcpA probe. Further, since the transcriptional activator ToxR is required for the optimal expression of genes under the control of ctx promoter the presence of toxR gene was ascertained by hybridization with the toxR probe.

The *V. cholerae* strain B0010 (ATCC 202010) was tested in ligated ileal loop assay as described by De S.N. in Nature 183, 1533-1534 (1949) and Formal et al in Br. J. Exp. Pathol. 42, 504-510 (1961). The strain was not capable of accumulating fluid in the ligated ileal loop rabbit model indicating that the strain does not produce any secretogenic factors.

The strain did not produce any adverse effect on rabbits when examined by the RITARD model following conventional methods showing that the strain is nonreactogenic. Despite non-reactogenicity, this strain displayed impressive colonization ability as determined by conventional method, which is required of the strain for it to function as effective vaccine.

The V. *cholerae* strain having the Accession No. B0010 (ATCC 202010) can be grown in Luria broth at 30-37°C temperature and stored in the same medium with 20% (vol/vol) glycerol at -80°C to -60°C.

To facilitate the preparation of the vaccine using the parent strain of *V. cholerae* having MTCC Accession No. B0010 (ATCC 202010), a series of genetic manipulations leading to the construction of ctx subunit gene flanked by hlyA gene sequences had to be carried out. These steps are given below
(i) Creation of a construct of *ctx*B subunit gene with its own SD sequence and the promoter of ctx-operon by first cloning the cholera toxin (ctx) operon from the chromosome of *V. chol*erae 569B strain (National Institute of Cholera and Enteric Diseases) and then deleting the *ctx*A subunit gene from the cloned operon by inverse PCR.
(ii) Cloning of the target locus hemolysinA gene (hlyA) for the purpose of targeted recombination.
(iii)Disruption of the hlyA gene sequence by the ctxB subunit gene construct as obtained at step (iii).
(iv) Cloning of the disrupted hlyA construct that has the ctxB subunit gene, into the suicide plasmid vector.
(v) Mobilisation of the suicide vector, bearing the disrupted hlyA construct into the parent strain [obtained at step (ii)] by conjugation, for targeted integration of the ctxB gene construct at the hly A gene.
(vi) Identification of the recombinant *V. cholerae* clones with the integrated ctxB gene by hybridization and polymerase chain reaction.
The first step among the manipulations was to clone the ctx operon of known *Vibrio cholerae.* The ctx operon of known *Vibrio cholerae* 01 strain 569B was cloned after its amplification from the genome by polymerase chain reaction. In the polymerase chain reaction, *Vibrio cholerae* 569B chromosomal DNA was used as template to amplify the ctx operon with the primers CT1 and CT2. Oligonucleotide CT1 (5' TTA GTG TTC GAT ACC TTT GCA 3') is complementary to the chromosomal DNA sequence located 149-172 nucleotides upstream of the transcription start site of ctx operon. Oligonucleotide CT2 (5' TTA GGC AAA ACG GTT GCT TCT TCT CAT CATC 3') is complementary to the chromosomal DNA sequence located 43-67 nucleotides down stream of the stop codon of ctx. When these two oligonucleotides are used as primers to amplify the DNA sequence, the product is the complete ctx operon consisting of the genes encoding cholera toxin A and B subunits along with the promoter and the upstream ToxR binding repeats at the 5' end, and the transcription termination signal at the 3' end of the operon. In the polymerase chain reaction, following an initial denaturation step the reaction was cycled about 30 times through a denaturation step, an annealing step and an extension step. At the end of the last cycle an additional extension step was included. After the polymerase chain reaction, the reaction mix was extracted with equal volume of 50:50 mixture of phenol : chloroform. The aqueous phase was passed through Sephadex G50 spun column to remove free nucleotides and precipitated by ethanol. After resuspending the DNA, it was treated with T4 DNA polymerase enzyme in the presence of dTTP. This resulted in the amplified DNA product with 5' TT-dinucleotide overhangs. To clone the amplified DNA that was modified as above, the plasmid vector pBS+ was first linearized with the restriction enzyme, EcoRI. It was then treated with the Klenow fragment of E.coli DNA polymerase I in presence of dATP. This treatment resulted in 5' AA overhangs at both the ends of the linearized pBS+ vector DNA. This vector DNA preparation was ligated to the amplified *ctx* operon with 5' TT-overhangs at 1:2 molar ratio by T4 DNA ligase. After this dinucleotide sticky end ligation the reaction mix was used to transform *E. coli* "Sure" strain by electroporation. The resulting recombinant plasmid clones were analysed by restriction enzymes for which the sites on the ctx operon were known (e.g. Nde I, XbaI, ClaI). The insert of one clone called pGT1 has the complete ctx operon as confirmed by restriction analysis and sequencing. The nucleotide sequence was determined to rule out the possibility of any error introduced during polymerase chain reaction. This plasmid clone pGT1 was used in the subsequent step. It has the complete ctx operon consisting of the genes encoding cholera toxin A and B subunits along with the promoter and the upstream ToxR binding repeats at the 5' end and the transcription termination signal at the 3' end of the operon.

In the next step, deletion of complete ctx-A coding sequence from the plasmid clone pGT1 was achieved by inverse polymerase chain reaction using oligonucleotide primers that diverge from each other and amplify the plasmid excluding the *ctx*-A gene. An inverse polymerase chain reaction was carried out using phosphorylated oligonucleotides CT3 and CT4 as primers and the plasmid pGT1 as the template DNA. The resulting amplified product was phenolysed, passed through Sephadex G-50 spun column to remove the free nucleotides and ethanol precipitated. It was then treated with T4 DNA polymerase in presence of dTTP to generate 5' GG-overhang at the terminus corresponding to CT3 and 5' CC-overhang at the terminus corresponding to CT4. Ligation of the termini which have the above dinucleotide overhangs complementary to each other, was carried out by T4 DNA ligase. The ligation product was used to transform *E. coli* Sure strain by electroporation. The recircularization of the inverse polymerase chain reaction product resulted in the fusion of the ctx promoter and the ctx B subunit gene. This construct ctx Promoter-B was identified in the various plasmid clones by polymerase chain reaction using CT1 and CT2 as primers as explained before which would yield a 0.63 kilobase fragment corresponding to the ctx promoter-B construct. In this ctx promoter-B construct the ctxB subunit gene is under the control of ctx operon promoter and its translation would be initiated at its own SD sequence. The expression of B subunit from the various clones was confirmed by Bead ELIZA assay. The nucleotide sequence of the *ctx*-Promoter-B construct of one plasmid clone pGT 3.1 was determined to analyse the sequence at the promoter-B subunit gene fusion point. The ctx Promoter-B gene construct from pGT3.1 was used in a latter step to disrupt cloned *hly* A gene.

Since it was decided to introduce the ctx Promoter-B gene construct into the *Vibrio cholerae* strain having the Accession No. B0010 (ATCC 202010) by targeted recombination at the *hly* A gene, it was essential to make a construct in which cloned *hly A* gene sequence is disrupted in the middle by the ctx Promoter-B construct. For this purpose it was necessary to clone the gene of *V. cholerae.* A partial *hly* A gene sequence which lacks the 5' flanking region and a large part of the coding region at the 3' side was cloned after its amplification from the *Vibrio cholerae* genome. The size of the complete coding region of *hly* A gene is about 2.2 kilobase out of which 1.7 kilobase of the *hly* A gene was amplified from the genomic DNA of *V. cholerae* of 01 serotype strain. In a polymerase chain reaction using appropriate oligo nucleotide primers after an initial denaturation step, the reaction was cycled through a denaturation step, an annealing step and an extension step. At the end of the last cycle an additional extension step was included. The polymerase chain reaction product was analysed by agarose gel electrophoresis for the presence of a 1.7 kilobase DNA fragment. The reaction product was phenolysed, passed through Sephadex G-50 spun column to remove free nucleotides and ethanol precipitated. The purified DNA was treated with T4 DNA polymerase in presence of dGTP to generate 5' TT-overhangs at the termini. To clone the amplified *hly* A gene sequence with the modified termini as above, the plasmid vector pUC9 was first linearized with the restriction enzyme EcoRI. The linearized pUC9 DNA was then treated with Klenow fragment of E.coli DNA polymerase I in presence of dATP to generate 5' AA-overhangs at the termini. This vector DNA preparation was ligated to the amplified *hly* A gene sequence with 5' TT overhangs by T4 DNA ligase. After the dinucleotide sticky end ligation the reaction mix was used to transform *E*. *coli* "Sure" strain by electroporation. The resulting plasmid clones were analysed by digestion with restriction endonucleases. One of the plasmid clones pGT89 which had the 1.7 kilobase *hly* A insert was used in the subsequent step to disrupt the hlyA sequence by ctx promoter-B gene construct.

In the next step the ctx Promoter-B construct of pGT 3.1 was amplified, using the oligonucleotide primers CT1 and CT2. The amplified product was phenolysed, passed through Sephadex G50 spun column and ethanol precipitated. The purified ctx Promoter-B construct fragment was treated with T4 DNA polymerase in presence of dTTP to generate 5' TT-overhangs at the termini. The ctx Promoter-B construct with 5' TT-overhangs was inserted into the middle of the cloned *hly* A gene sequence in place of the 0.4 kilobase HpaI fragment. Since the plasmid vector pUC9 does not have a site for HpaI enzyme, digestion of pGT89 with HpaI would result in a 0.4 kilobase fragment from the middle of the cloned *hly* A gene sequence and a 3.9 kilobase fragment. pGT89 was digested with HpaI enzyme and the DNA fragments were separated by agarose gel electrophoresis. The 3.9 kilobase HpaI fragment was electroeluted and purified. It was then treated with T4 DNA polymerase in presence of dGTP to generate 5' AA-overhangs at the termini. The 3.9 kilobase fragment with 5' AA-overhangs was ligated to the ctx Pr-B construct with 5' TT-overhangs by T4 DNA ligase. The ligated mix was used to transform *E*. *coli* "Sure" strain by electroporation. The recombinant plasmid clones were identified by colony hybridization using ctx Promoter-B construct as the probe. Digestion of the recombinant plasmids with EcoRI enzyme gave an 1.9 kilobase insert of *hly* A gene sequence disrupted in the middle by the ctx Promoter-B construct. The EcoRI insert from one recombinant plasmid pGT 39 was used for cloning it into the suicide vector pGP 704 (Miller V.L. and J.J. Mekalanos (1988) J Bac 170; 2575-2583).

In the next step the ctx promoter-B construct along with the flanking hlyA sequences was cloned into the plasmid suicide vector. The plasmid suicide vector pGP704 was digested with EcoRI enzyme and the 5' phosphate groups of the linearized vector were removed by treatment with calf intestinal phosphatase. The EcoRI digested, dephosphorylated pGP704 was electrophoresed on a 0.8% agarose gel and the corresponding DNA band was electroeluted and purified. This was done to make sure that no trace of the undigested vector DNA was present in the vector DNA preparation. It was then ligated to the 1.9 kilobase EcoRI insert of pGT39 at 1:5 molar ratio by T4 DNA ligase and the ligated mix was used to transform *E coli* SM10 pir strain by electroporation. The recombinant plasmid clones were identified by colony hybridization, using ctx Promoter-B construct as the probe. Digestion of the recombinant plasmid with EcoRI gave 1.9 kilobase insert which represented the cloned hlyA gene sequence disrupted by the ctx Promoter-B construct. One of the clones pGT27 was used for mobilisation into the *V. cholerae* strain having Accession No. B0010 (ATCC 202010) for integration at the hlyA gene.

In the following step mobilization of pGT27 into V. *cholerae* 01 strain having Accession No. B0010 (ATCC 202010) was achieved by conjugation between the *Vibrio cholerae* having Accession No. B0010 (ATCC 202010) strain and the *E. coli* strain SM10 pir containing the plasmid pGT27. The plasmid clone pGT27 in the E. *coli* host strain SM10 pir and the V. *cholerae* strain having Accession No. B0010 (ATC 202010) were grown in LB to midlog phase without shaking. A small quantity of the donor strain i.e. SM10 pir strain with pGT27 and an equal quantity of the recipient strain i.e V. *cholerae* having Accession No. B0010 (ATCC 202010) strain were mixed in 1.5ml microfuge tubes and briefly centrifuged to collect the cells at the bottom. After removing the supernatant Luria broth was added to cell pellet and the cells were suspended in it by pipetting up and down. The cell suspension was then spotted on a 0.4mm membrane disc placed on the surface of a LB agar medium. The suspension soon got absorbed on the membrane. The donor and the recipient cells on the membrane were allowed to mate for about 4-9 hours at 37°C . The membrane filter was then collected into a tube and 1ml of Luria broth was added. The cells on the membrane were resuspended in the medium by vortexing briefly. The suspension so obtained was spread on LB agar medium containing 10µg/ml of streptomycin sulfate and an appropriate concentration of ampicillin, to select only the recombinant V. *cholerae* having Accession No. B0010 (ATCC 202010) clones which had the plasmid pGT27 integrated into their genome. (The ampicillin resistance is conferred by the suicide plasmid vector). When the sensitivity of the parent *V. cholerae* having Accession No. B0010 (ATCC 202010) strain to ampicillin was checked, it was found that an ampicillin concentration as low as 5ug/ml, could completely inhibit its growth on LB agar medium. So selection of recombinant clones in which pGT27 got integrated, was performed on LB plates containing 10ug/ml of streptomycin sulfate and 10-100ug/ml of ampicillin.

The recombinant *V. cholerae* clones were identified by colony hybridization followed by the colonies that appeared on the above plates were analysed by colony hybridization with the ctx B gene sequence as the probe. Since the recipient V. *cholerae* having Accession No. B0010 (ATCC 202010) does not possess the ctx operon and the ctx Promoter-B construct was introduced through pGT27, only the recombinant *V. cholerae* clones in which the pGT27 got integrated were expected to hybridize with the ctx B gene probe. All the ampicillin resistant colonies hybridized to the ctx B probe. From several colonies, chromosomal DNA was isolated and used as template DNA in separate polymerase chain reactions with the hly A specific primers HA1 and HA2. These two primers define a 1.7 kilobase sequence from wild type hlyA gene and 1.9 kilobase sequence from pGT27 in which the hlyA gene sequence is disrupted by ctx Promoter-B construct. When *V. cholerae* having Accession No. B0010 (ATCC 202010) chromosomal DNA is used as a template, these primers will amplify only the 1.7 kilobase sequence, whereas when the chromosomal DNA of recombinant *V. cholerae* is used as template these primers will amplify a 1.9 kilobase fragment besides the 1.7 kilobase fragment. In all the clones tested by polymerase chain reaction both the above mentioned fragments got amplified by the primers HA1 and HA2. The expression of ctxB subunit from these clones was analysed by bead ELIZA, as described in the article "Detection of cholera toxin by a highly sensitive bead-Enzyme Linked Immuno Zorbent Assay" by Yoshihiko Uesaka, Yoko Otsuka, Miksuaki Ka hida, Yuichi Oku, Kazuki Horigome, G. Balakrish Nair, S.C: Pal, Shinji Yamasaki and Yoshifumi Takeda in Microbiol. Immunology, Vol. 36(1), 43-53, (1992).

In order to perform genomic Southern hybridization, genomic DNA isolated from the recombinant *V. cholerae* clones was digested with XbaI, SalI and HpaI separately. The DNA fragments of individual restriction digestions were separated by electrophoresis on agarose gel and transferred and immobilised on to a nylon membrane by Southern transfer. The immobilized DNA was hybridized with ctx B gene probe and hly A probe in separate experiments. The analysis of the genomic Southern hybridization results revealed that pGT27 indeed got integrated at the hlyA gene of V. *cholerae* having Accession No. B0010. It also revealed that pGT27 is repeated in tandem in several recombinant clones. One of the clones, was chosen as vaccine strain and tested in animal models. The cholera vaccine strain has been deposited at Microbial Type Culture Collection, Institute of Microbial Technology, Chandigarh, India, and has been given the Accession No. B0011 (ATCC 202011). The strain can be grown in Luria broth medium at 30-37°C temperature and stored in the same medium with 20% (vol/vol) glycerol at -80 to -60°C. The ability to produce the B subunit of cholera toxin by the vaccine strain which has the Accession No. B0011 (ATCC 202011) was monitored using the bead-ELIZA by Yoshihiko Uesaka, Yoko Otsuka, Miksuaki Ka hida, Yuichi Oku, Kazuki Horigome, G. Balakrish Nair, S.C. Pal, Shinji Yamasaki and Yoshifumi Takeda in Microbiol. Immunology, Vol. 36(1), 43-53, (1992). The parent strain (MTCC Accession No. B0010) did not produce B subunit whereas the vaccine strain has the Accession No. B0011 (ATCC 202011) showed copious production of the B subunit. The amount of B subunit produced by this strain having the Accession No. B0011 (ATCC 202011) was several fold higher than the strain having the Accession No. B0010 (ATCC 202010) as reflected by the higher optical density because the former had multiple copies of CT-B as compared to the latter which had only a single copy. Having determined that the genetic manipulations were appropriately done, the next effort made was to determine whether the vaccine induced fluid accumulation in the ligated rabbit ileal loop assay . Ligated ileal loop assay (rabbit model) was carried out as described in De, S.N., Nature 183, 1533-1534 (1959) and Formal, S.B., Kundel, D., Schneider, H., Kunev, N. and Sprinz, H. Br. J. Exp. Pathol. 42, 504-510 (1961). The vaccine strain having the Accession No. B0011 (ATCC 202011) did not induce fluid accumulation. This attested the fact that the vaccine construct produces the B subunit but as is expected this subunit is inocuous and incapable of inducing fluid accumulation. The next series of experiments were conducted to determine whether the vaccine strain was reactogenic using an in vivo rabbit model. Vaccine trials on rabbits by RITARD model were performed as described by Spira, N.M., Sack, R.B. and Froehlich, J.L. (1981). Infect. Immun, 32, 739-747. It was clear that the vaccine strain having the Accession No. B0011 (ATCC 202011) did not induce diarrhoea when fed orally to rabbits. Based on these results challenge studies with strains of *V. cholerae* of 01 serotype belonging to both E1 Tor and classical biotypes were conducted in several batches. The data of the challenged studies in rabbits can be summarised as follows
1. Rabbit immunized with vaccine strain having the Accession No. B0011 (ATC 202011) were significantly protected from a challenge with a classical biotype and an ElTor biotype strain. This was evident from the absence of diarrhoea in the immunized rabbits while the controls rabbits had profound diarrhoea.
2. The colonization ability of the challenge strains in the immunized rabbits were significantly lower as compared to the control rabbit.
3. The non-reactogenicity of the vaccine strain having the Accession No. B0011 (ATCC 202011) was again evident by its inability to provoke diarrhoea in the control rabbits.
4. The immunogenicity of the vaccine strain having the Accession No. B0011 (ATCC 202011) was evident since there was a significant rise in antibody titre against lipopolysaccharide, outer membrane protein, whole cell lysate and cholera toxin in immune sera as compared to the preimmune sera of rabbits orally immunized with the vaccine strain.

All the experimental procedures described are carried out following standard methods and experimental conditions given below :
*Escherichia coli* and *Vibrio cholerae* strains were grown and maintained in Luria Broth (LB). Where required, appropriate antibiotics were added to the growth medium. Preparation of ampicillin, streptomycin sulfate stocks, LB medium and other details for handling bacteria were followed from Molecular cloning (1989) by J. Sambrook, E.F. Fritsch and T. Maniatis, Cold Spring Harbour Laboratory.

The polymerase chain reactions were carried out using vent DNA polymerase (exonuclease⁺) and the basic reaction conditions were 10mM KCl, 10mM (NH₄)₂ SO₄, 20mM Tris Hcl (pH 8.8), 4mM MgSO4, 200uM each dNTP, 0.1% Triton X-100, lum primers, 100 ng of chromosomal DNA template or 10-20ng of plasmid DNA template and 2 units of vent DNA polymerase in a total volume of 100ul.

Treatment of DNA with T4 DNA polymerase was carried out in 33mM Tris acetate (pH 8.0), 66mM potassium acetate, 10mM magnesium acetate, 0.5mM dithiothreitol, 100ug/ml bovine serum albumin (BSA), 2 units of T4 DNA polymerase, at 12°C for 30 minutes in presence of appropriate dNTP.

Treatment of DNA with Klenow fragment of *E. coli* DNA polymerase I was carried out in 50mM Tris-cl (pH 7.6), 10mM mgcl₂, 50ug/ml BSA and 1 unit of the enzyme at room temperature for 20 minutes, in presence of appropriate dNTP.

Ligation of the DNA was carried out in 20mM Tris-C! (pH 7.6), 5mM Mgc12, 5mM DTT 50ug/ml BSA, 1.5 unit of T4 DNA ligase and 0.5mM ATP at 12°C for 12-16 hours.

Transformation of *E. coli* cells was performed by electroporation using BIORAD gene pulser. Preparation of *E. coli* cells or electroporation and the electroporation details were followed from manufacturer's instructions.

Dideoxy nucleotide sequencing of DNA was performed using the sequenase kit obtained from the United States Biochemicals company, Cleveland, Ohio, USA.

Procedures for isolating plasmid DNA, digestion of DNA with restriction enzymes, agarose gel electrophoresis of DNA, electroelution of DNA fragments, purification of DNA by phenol extraction, spun column chromatography in sephadex G-50 to remove free nucleotides from the polymerase chain reactions, precipitation of DNA with ethanol, radiolabelling of DNA probe, colony hybridization and Southern hybridization protocols are all essentially as described in Molecular cloning (1989) by J. Sambrook, E.F. Fritsch, and T. Maniatis, Cold Spring Harbour. Protocol to isolate genomic DNA from *Vibrio cholerae* was followed from Current Protocols in Molecular Biology by Ausubel et al (1987) Massachusetts General Hospital & Harvard Medical School.

The process of the present invention is illustrated through the examples given below, which should not however be construed to limit the scope of the present invention.

### Example 1 :

### Isolation of the parent V. cholerae strain having MTCC accession no. B0010

Stool specimens from cholera patients were collected immediately on admission to the Infectious Diseases Hospital, Calcutta in sterile MacCartney bottles with sterile catheters. Soon after collection, stool specimens were transported to the laboratory and examined within 2 h for *V*. *cholerae* and for other common toxinogenic enteropathogens such as enterotoxinogenic *E. coli* (ETEC), Shigella, Salmonella and Campylobacter spp. by standard published techniques (World Health Organization. Programme for control of diarrhoeal diseases (CCD/83.3 Rev. 1], Manual for laboratory investigations of acute enteric infections. Geneva, World Health Organization 1987). To identify *V*. *choler*ae strains in the stool specimens, plates of Thiosulphate-Citrate-Bile Salts-Sucrose Agar (TCBS; Eiken, Japan) were streaked with two or three loopfuls of stool specimens for selective isolation of *V*. *cholerae.* After incubation overnight at 37°C, colonies from each stool sample showing growth on the selective agar were inoculated on to a multi-test medium (Nair GB, Misra S, Bhadra RK, Pal SC. Appl Environ Microbiol 1987, 53 : 1203-1205). Strains that showed typical alkaline slant-acid butt reaction on the multi-test medium were examined for the oxidase reaction and slide agglutination was performed with polyvalent 01 and monospecific Ogawa-Inaba antisera prepared at the National Institute of Cholera and Enteric Diseases, Calcutta, India. Several hundred strains of V. *cholerae* O1 obtained as above were plated as colonies on Luriabroth agar for screening by hybridization with DNA probes specific for toxin genes ctx, zot and ace. Purified DNA was labelled with ³²P by incorporating [α-^{32p}] dATP to a specific activity of 2x10⁸-8x10⁸ cpm/µg of DNA by nick translation. Radiolabelled probe DNA was purified by chromatography on NACS PREPAC as specified by the manufacturer (Bethesda Research Laboratories, USA). The colony blot was prepared on autoclaved gridded nitrocellulose filter (Schleicher and Schuell Co.; BA 85/20 and hybridisation was performed under high stringency as described previously (ref. Karasawa T, Mihara T, Kurazono H et al. Distribution of the zot (zonula occludens toxin) gene among strains of *Vibrio cholerae* O1 and non-O1. FEMS Microbiol Lett 1993; 106: 143-146).

The strains that were found to be devoid of the virulence genes viz. ctx, zot and ace were tested for cholera toxin like and other cytotoxin activities. For this purpose the strains were grown in casaminoacid yeast extract (CAYE) medium supplemented with 90µg/ml lincomycin and the culture filtrates were examined for the presence of cholera toxin (T)-like enterotoxin and for thermostable direct haemolysin in a highly sensitive bead-ELIZA (ref. Oku Y, Uesaka Y, Hirayama T, Takeda Y. Development of a highly sensitive Bead-ELIZA to detect bacterial protein toxins. Microbiol Immumol 1988; 32: 807-816; Uesaka Y, Otsuka Y, Kashida M et al. Detection of cholera toxin by a highly sensitive bead-enzyme linked immunosorbent assay. Microbiol Immunmol 1992; 36: 43-53). Various dilutions of purified CT (Sigma) or the culture filtrate of strain NICED 10 of Kanagawa phenomenon-positive *V. parahaemolyticus* (positive controls) and uninoculated medium (negative control) were run concurrently whenever a batch of the culture filtrate of the test strains were assayed by the bead-ELIZA. Haemolytic activity of the parent strain V. *cholerae* having the accession no. B0010 with erythrocytes from rabbit, sheep, chicken and man was determined as described previously (ref. Nair GB, Oku Y, Takeda Y et al. Toxin profiles of *Vibrio cholerae* non-O1 from environmental sources in Calcutta, India. Appl Environ Microbiol 1988; 54: 3180-3102). One of the strains which does not have the virulence genes and which does not have cholera toxin like and other cytotoxin activities has been given the Accession No. B0010(ATCC 202010). In this strain the presence of the cryptic hemolysin A gene has been ascertained by hybridization with hlyA probe. This is essential since the integration of ctxB subunit gene has to be carried out by targeted recombination at hlyA locus. Since the product of tcpA gene is the main component of the pilus required for effective colonization of *Vibrio cholerae* in the intestine, its presence has also been ascertained by hybridization with tcpA probe. Further, since the transcriptional activator ToxR is required for the optimal expression of genes under the control of ctx promoter the presence of the *tox*R gene has been ascertained by hybridization with the *tox*R probe.

Further the *V. cholerae* strain having the Accession No. B0010 (ATCC 202010) has been tested in ligated ileal loop assay and RITARD model to check if it is reactogenic and whether it can colonize the intestine effectively. For rabbit ileal loop assays outbred New Zealand white rabbits of either sex weighing between 1.5 to 2.5 kg body weight were selected. Experimental animals were starved for 36 hours but were given water ad libitum. Surgery was done under anaesthesia given intravenously. The ileal loop procedure was essentially similar to that described by S.N. De [De, S.N., Nature, 183, 1533-1534 (1959)]. The intestine was brought out through a midline incision. The selected small intestinal portion were washed carefully with warm (37°C) sterile PBS (0.1 M, pH 7.4). A total of 8 loops were prepared in each rabbit. The length of the loop and the inter loop were 5 cms and 2 cms, respectively. One ml of live bacterial cell suspension containing about 10⁸ cells was then introduced in each loop. *V. cholerae* 569B was used as the positive control and sterile PBS (0.01 M, pH 7.4) was used as the negative control. After inoculation, the small intestine of the animal was introduced carefully inside the open abdomen and then the incision was sutured. Animals were kept in their cages and supplied with water. Animals were sacrificed after 18 to 20 hours and an index of fluid accumulation (FA) was calculated from the ratio of loop fluid volume to loop length which was expressed as ml/cm. A test preparation was considered positive if the ratio was > 0.9. Results were discarded if control reactions were inappropriate. The V. *cholerae* strain having the Accession No. B0010 (ATCC 202010) was tested twice in the ileum of two different rabbits. It did not cause any fluid accumulation. The strain having the Accession No. B0010 (ATCC 2020210) was tested for its diarrhoeagenicity and colonization ability in the RITARD model. For this purpose outbred New Zealand white rabbits of either sex weighing between 1.7 to 2.5 kg were selected for the colonization experiments. All the animals were acclamatized in the laboratory for a week. The experimental rabbits were treated with a course of metronidazole (125 mg/rabbit/day) and sulfaquinoxaline sodium (464 mg/rabbit/day) and this course was repeated at an interval of two days to clean the animal of intestinal protozoan pathogens like Giardia and Coccidia. For preparing the oral inoculum the strain having the accession no. B0010 was grown overnight in tryptic soy broth (TSB, Difco, USA) at 37°C for 18 hours in an orbital shaker. Cells were harvested by centrifugation at 8000 rpm for 15 minutes. The pellet was suspended in sterile phosphate buffered saline (pH 7.4) and the bacterial density was estimated in a spectrophotometer at 540 nm and diluted using PBS to an optical density of approximately 10⁹ cells for use as the inoculum. For oral inoculation rabbits were fasted for 36 hours but water was given adlibitum; 35 minutes before oral inoculation each rabbit was anesthetized intramuscularly with ketamine (35 mg/kg body weight) and 4 mg/kg body weight of xylazine. After 5 minutes, the rabbits were administered 50 mg of cimetidine which is a H₂ receptor blocker and inhibits the secretion of HC1 from peptic cells of the stomach. After 15 minutes a feeding tube (Accumark, Feeding Catheter, USA) was placed per os and 15 ml of a 5% solution of sodium bicarbonate (SRL India, sodium bicarbonate neutralizes HC1 present in stomach) was introduced. At '0' time another 15 ml of 5% solution of sodium bicarbonate was given followed immediately by the bacterial inoculum suspended in 15 ml of 0.01 M PBS (pH-7.4). After 30 minutes, 2 ml of tincture of opium was given intraperitoneally. The rabbits were then returned to the cages and given limited amount of sterilized water and food. Diarrhoea was scored according to a grading system using the following characteristics. Stools were graded as follows : grade 1 represents normal stool without diarrhoea; grade 2 represents diarrhoea with soft mushy stools also termed as moderate diarrhoea and grade 3 represents diarrhoea with catarrhal and watery diarrhoea being termed as severe diarrhoea. The strain having the accession no. B0010 does not cause diarrhoea showing it is not reactogenic. To study the colonization of the *V. cholerae* strain having the accession no. B0010 the experimental rabbits were sacrificed after 18 hours of inoculation. The rabbits were anesthetized and the intestine was taken out after opening the abdomen surgically. Ten cm of the distal ileum was tied at both ends with umbilical tape (No. 11) and cut. The 10 centimeter ileum was placed into a beaker containing 10 ml of sterile 0.01 M PBS (pH 7.4). The pieces of intestine were opened longitudinally and washed gently. Serial dilutions were prepared from the washed materials. The tissue portion of ileum was weighed and homogenized with 10 ml of PBS (0.01 M, pH 7.4) and homogenized. Serial dilutions of the homogenate was prepared. The rabbits were finally killed using 2 ml of Euthanasia 6-solution 1 (Veterinary Lab./Inc. Kansas, USA) by intravenous injection. Neat and serial dilutions of the intestinal wash and homogenized material of ileum was plated on selective medium. The plates were incubated at 37°C and colony counts were made 24 hours later using a colony counter and expressed as colony forming units. The *V. cholerae* strain having the accession no. B0010 displays impressive colonization ability. This is probably due to the presence of the toxin coregulated pilus whose presence was determined by hybridization.

The V. *cholerae* strain having the Accession No. B0010 (ATCC 20201) can be grown in Luria broth at 37°C temperature and stored in the same medium with 20% (vol/vol) glycerol at -70°C. This strain having the Accession No. B0010 (ATCC 202010) has been used as parent strain for preparation of the cholera vaccine strain having the accession no. B0011.

### Example 2 :

The vaccine strain of *Vibrio cholerae,* having the Accession No. B0011 (ATCC 202011) has been constructed by integrating the gene encoding the B subunit of the cholera toxin into the chromosome of the parent strain having the Accession No. B0010 (ATCC 202010). This has been achieved by targeted recombination, at the hlyA locus of the chromosome of the *V. cholerae* strain having Accession No. B0010 (ATCC 202010). This briefly involves the following steps :
(i) Creation of a construct of ctxB subunit gene with its own SD sequence and the promoter of ctx-operon by first cloning the cholera toxin (ctx) operon from the chromosome of known *V. cholerae* 569B strain (National Institute of Cholera and Enteric Diseases) and then deleting the *ctx*A subunit gene from the cloned operon by inverse PCR.
(ii) Cloning of the target locus hemolysinA gene (hlyA) for the purpose of targeted recombination.
(iii)Disruption of the hlyA gene sequence by the ctxB subunit gene construct as obtained at step (iii).
(iv) Cloning of the disrupted hlyA construct that has the ctxB subunit gene, into the suicide plasmid vector.
(v) Mobilisation of the suicide vector, bearing the disrupted hlyA construct into the parent strain [obtained at step (ii)] by conjugation, for targeted integration of the ctxB gene construct at the hly A gene.
(vi) Identification of the recombinant *V. cholerae* clones with the integrated ctxB gene by hybridization and polymerase chain reaction.

To facilitate the preparation of the vaccine using the parent strain, having MTCC Accession No. B0010 (ATCC 202010) a series of genetic manipulations, leading to the preparation of ctxB subunit gene flanked by hlyA gene sequences, were carried out. The first step among the manipulations was to clone the ctx operon of *Vibrio cholerae.* The ctx operon of *Vibrio cholerae* strain 569B was cloned after its amplification from the genome by polymerase chain reaction. For the amplification of ctx operon the following primers were used. Oligonucleotide CT1 (5' .TTA GTG TTC GAT ACC TTT GCA 3') was complementary to the chromosomal DNA sequence located 149-172 nucleotides upstream of the transcription start site of ctx operon. Oligonucleotide CT2 (5' TTA GGC AAA ACG GTT GCT TCT TCT CAT CATC 3') was complementary to the chromosomal DNA sequence located 43-67 nucleotides down stream of the stop codon of ctx. When these two oligonucleotides were used as primers to amplify the DNA sequence, the product was the complete ctx operon consisting of the genes encoding cholera toxin A and B subunits along with the promoter and the upstream ToxR binding repeats at the 5' end, and the transcription termination signal at the 3' end of the operon. In the polymerase chain reaction 100 ng of the known *Vibrio cholerae* 569B chromosomal DNA was used as template to amplify the ctx operon with 1.0uM of the primers CT1 and CT2. Following an initial denaturation step at 97°C for 2 minutes the reaction was cycled 30 times through a denaturation step at 94°C for 1 minute, annealing step at 62°C for 1 minute and extension step at 72°C for 2 minutes. At the end of the last cycle an additional extension step was included for 10 minutes at 72°C. After the polymerase chain reaction, the reaction mix was extracted with equal volume of 50:50 mixture of phenol : chloroform. The aqueous phase was passed through Sephadex G50 spun column to remove free nucleotides and precipitated by ethanol. After resuspending the DNA, it was treated with T4 DNA polymerase enzyme in the presence of 200uM dTTP. This resulted in the amplified DNA product with 5' TT-dinucleotide overhangs. To clone the amplified DNA that was modified as above, the plasmid vector pBS+ was first linearized with the restriction enzyme, EcoRI. It was then treated with the Klenow fragment of E.coli DNA polymerase I in presence of 200 uM dATP. This treatment resulted in 5' AA overhangs at both the code of the linearized vector DNA. This vector DNA prepare tion was ligated to the amplified ctx operon with 5' TT-overhangs at 1:2 molar ratio by T4 DNA ligase. After this dinucleotide sticky end ligation the reaction mix was used to transform *E*. *coli* "Sure" strain by electroporation. The resulting recombinant plasmid clones were analysed by restriction enzymes for which the sites on the ctx operon were known (e.g. Nde I, XbaI, ClaI). The insert of one clone called pGT1 had the complete ctx operon as confirmed by restriction analysis and sequencing. The nucleotide sequence of the complete B subunit gene was determined to rule out the possibility of any error introduced during polymerase chain reaction. This plasmid clone pGT1 was used in the subsequent step.

In the next step deletion of complete ctx-A coding sequence from the plasmid clone pGT1 was achieved by inverse polymerase chain reaction using oligonucleotide primers that diverge from each other and amplify the plasmid excluding the ctx-A gene. Oligonucleotide CT 3 (5' CCA TTG TTT AAC AGA AAA ATA ATT GAT CAA AAC 3') was complementary to the chromosomal DNA sequence located +9 to -21 nucleotides with respect to the transcription start site of the ctx operon. Oligo nucleotide CT4 (5' GGA ATT AAG GAT GAA TTA TGA TTA AAT TAA AA-3') was located -16 to +15 nucleotides with respect to the first base of the initiation codon of ctx B gene. The oligonucleotides CT3 and CT4 were phosphorylated at their 5' ends by T4 polynucleotide kinase. An inverse polymerase chain reaction is carried out using these phosphorylated oligonucleotides CT3 and CT4 as primers and the plasmid pGT1 as the template DNA. The resulting amplified product which was 3.8 kilobase in size, is phenolysed, passed through Sephadex G-50 spun column to remove the free nucleotides and ethanol precipitated. It was then treated with T4 DNA polymerase in presence of 200 uM dTTP to generate 5' GG-overhang at the terminus corresponding to CT3 and 5' CC-overhang at the terminus corresponding to CT4. Ligation of the termini which had the above dinucleotide overhangs complementary to each other, was carried out by T4 DNA ligase at a final concentration of 2ng/ul of the DNA in the ligation mix, to facilitate intramolecular ligation. The ligation product was used to transform *E. coli* Sure strain by electroporation. The recircularization of the inverse polymerase chain reaction product resulted in the fusion of the ctx promoter and the ctx B subunit gene. This construct ctx Promoter-B was identified in the various plasmid clones by polymerase chain reaction using CT1 and CT2 as primers which yielded a 0.63 kilobase fragment corresponding to the ctx Promoter-B construct. The expression of B subunit from the various clones was confirmed by Bead ELIZA assay. The nucleotide sequence of the ctx-Promoter-B construct of one plasmid cloue pGT 3.1 was determined to analyse the sequence at the promoter-B subunit gene fusion point. This confirmed the fusion of the ctx operon promoter and the ToxR binding repeats with the ctxB subunit gene containing its own SD sequence. The ctxB gene exhibited a theoretically perfect SD sequence (TAA GGA) and there is evidence in the literature that the *ctxB* ribosome binding site is about nine fold more efficient than the *ctx*A site. (J.J. Mekalanos, D.J. Swartz G.D.N. Pearson, Nitarford, I. Groyne & Michel de Wilde (1983) Nature 306, 551 - 557). The *ctx* Promoter-B gene construct from pGT3.1 was used in a latter step to disrupt cloned *hly* A gene.

Since it was decided to introduce the ctx Promoter-B. gene construct into the *Vibrio cholerae* strain having the Accession No. B0010 (202010) by targeted recombination at the *hly* A gene, it was essential to make a construct in which cloned *hly* A gene sequence is disrupted in the middle by the ctx Promoter-B construct. For this purpose it was necessary to clone the *hly*A gene of *V. cholerae.* A partial *hly* A gene sequence which lacks the 5' flanking region and a large part of the coding region at the 3' side was cloned after its amplification from the *Vibrio cholerae* genome. For the amplification, the following oligo nucleotide primers were used. Oligo nucleotide HA1 (5' TTC ACA GAG TCA GTG AGG TTT ATA TGC C-3') was complementary to the chromosomal DNA sequence located between -19 to +6 nucleotides with respect to the start codon of the *hly* A gene and oligo nucleotide HA2 (5' TTC GCT GTA GAC ATT GGT CAA TTC ATC 3') was complementary to the chromosomal DNA sequence located between 1714-1690 nucleotides from the start codon of the *hly* A gene. The size of the completé coding region of *hly* A gene is about 2.2 kilobase out of which 1.7 kilobase of the *hly* A gene was amplified from the genomic DNA of *V. cholerae* O1 strain. After an initial denaturation step at 97°C for 2 minutes, the reaction was cycled 30 times through a denaturation step at 94°C for 1 minute, an annealing step at 62°C for 1 minute and an extension step at 72°C for 2 minute. At the end of the last cycle an additional extension step was included at 72°C for 10 minutes. The polymerase chain reaction product was analysed by agarose gel electrophoresis for the presence of a 1.7 kilobase DNA fragment. The reaction product was phenolysed, passed through Sephadex G-50 spun column to remove free nucleotides and ethanol precipitated. The purified DNA was treated with T4 DNA polymerase in presence of 200µM dGTP to generate 5' TT-overhangs at the termini. To clone the amplified *hly* A gene sequence with the modified termini as above, the plasmid vector pUC9 was first linearized with the restriction enzyme EcoRI. The linearized pUC9 DNA was then treated with Klenow fragment of E.coli DNA polymerase I in presence of 200µM dATP to generate 5' AA-overhangs at the termini. This vector DNA preparation was ligated to the amplified lily A gene sequence with 5' TT overhangs at 1:2 molar ratio by T4 DNA ligase. After the dinucleotide sticky end ligation the reaction mix was used to transform *E. coli* "Sure" strain by electroporation. The resulting plasmid clones were analysed by digestion with EcoRI and HpaI restriction enzymes. The 1.7 kilobase EcoRI insert should have 2 HpaI sites in the middle region separated by a 0.4 kilobase sequence. One of the plasmid clones pGT89 which had the 1.7 kilobase *hly* A insert was used in the subsequent step to disrupt the hlyA sequence by ctx promoter-B gene construct.

In the next step the ctx Promoter-B construct of pGT 3.1 was amplified, using the oligonucleotide primers CT1 and CT2. The amplified product was phenolysed, passed through Sephadex G50 spun column and ethanol precipitated. The purified ctx Promoter-B construct fragment was treated with T4 DNA polymerase in presence of dTTP to generate 5' TT-overhangs at the termini. The ctx Promoter-B construct with 5' TT-overhangs had to be inserted into the middle of the cloned *hly* A gene sequence in place of the 0.4 kilobase HpaI fragment. Since the plasmid vector pUC9 does not have a site for HpaI enzyme, digestion of pGT89 with HpaI should result in a 0.4 kilobase fragment from the middle of the cloned *hly* A gene sequence and a 3.9 kilobase fragment. pGT89 was digested with HpaI enzyme and the DNA fragments were separated by agarose gel electrophoresis. The 3.9 kilobase HpaI fragment was electroeluted and purified. It was then treated with T4 DNA polymerase in presence of dGTP to generate 5' AA-overhangs at the termini. The 3.9 kilobase fragment with 5' AA-overhangs was ligated to the ctx Pr-B construct with 5' TT-overhangs by T4 DNA ligase. The ligated mix was used to transform *E. coli* "Sure" strain by electroporation. The recombinant plasmid clones have been identified by colony hybridization using ctx Promoter-B construct as the probe. Digestion of the recombinant plasmids with EcoRI enzyme resulted in an 1.9 kilobase insert of *hly* A gene sequence disrupted in the middle by the ctx Promoter-B construct. The EcoRI insert from one recombinant plasmid pGT 39 was used for cloning it into the suicide vector pGP 704. The nucleotide sequence of the ctx Promoter-B construct along with the flanking hly A sequences of pGT39 was determined.

In the next step the ctx promoter-B construct along with the flanking hly A sequences was cloned into the plasmid suicide vector. The plasmid suicide vector pGP704 was digested with EcoRI enzyme and the 5' phosphate groups of the linearized vector were removed by treatment with calf intestinal phosphatase. The EcoRI digested, dephosphorylated pGP704 was electrophoresed on a 0.8% agarose gel and the corresponding DNA band was electroeluted and purified. This was done to make sure that no trace of the undigested vector DNA was present in the vector DNA preparation. It was then ligated to the 1.9 kilobase EcoRI insert of pGT39 at 1:5 molar ratio by T4 DNA ligase and the ligated mix was used to transform *E.coli* SM10 pir strain by electroporation. The recombinant plasmid clones were identified by colony hybridization, using *ctx* Promoter-B construct as the probe. Digestion of the recombinant plasmid with EcoRI gave 1.9 kilobase insert which represented the cloned hlyA gene sequence disrupted by the ctx Promoter-B construct. One of the clones pGT27 was used for mobilisation into the V. *cholerae* strain having the Accession No. B0010 (202010) for integration at the hlyA gene.

In the following step mobilization of pGT27 into parent *V. cholerae* strain having the Accession No. B0010 (ATCC 202010) was achieved by conjugation between the *Vibrio cholerae* strain having the accession no. B0010 and the *E. coli* strain SM10 pir containing the plasmid pGT27. The plasmid clone pGT27 in the *E. coli* host strain SM10 pir and the *V. cholerae* strain having the accession No. B0010 (ATCC 202010) were grown in Luria broth to midlog phase without shaking at 37°C. 0.5 ml of the donor strain i.e. SM10 pir strain with pGT27 and 0.5ml of the recipient strain i.e V. *cholerae* strain having the Accession No. B0010 (202010) were mixed in 1.5ml microfuge tubes and briefly centrifuged at 6000 rpm for 1 minute to collect the cells at the bottom. After removing the supernatant 50µl volume of Luria broth was added to cell pellet and the cells are suspended in it by pipetting up and down. The cell suspension was then spotted on a 0.4mm membrane disc placed on the surface of a LB agar medium. The suspension soon got absorbed on the membrane. The donor and the recipient cells on the membrane were allowed to mate at 37°C for 4 hours. The membrane filter was then collected into a 5ml screw capped tube and 1ml of LB medium was added. The cells on the membrane were resuspended in the medium by vortexing briefly. 10µl of the cell suspension was spread on LB agar medium containing 10µg/ml of streptomycin sulfate and 100ug/ml of ampicillin. At such a high concentration of ampicillin it was expected that the plasmid pGT27, which confers the ampicillin resistance, might get repeated in tandem at a relatively higher frequency and get selected because of the drug pressure. The ampicillin resistant colonies were analysed by hybridization with ctxB gene probe. The chromosomal preparations DNA from the recombinant clones that hybridized with the probe, were used as template in polymerase chain reaction with the *hly* A specific primers HA1 and HA2. From all the clones both the 1.7 kilobase and the 1.9 kilobase fragments were amplified. When two different size fragments defined by the same set of primers as in this case, were present in single copy then the smaller one was usually preferentially amplified in the polymerase chain reaction. This was reflected in their band intensities when examined by agarose gel electrophoresis. If the larger one was present in multiple copies then the amount of larger fragment amplified would be much more than when it was present as a single copy.

One of the clones which was found to have more intense 1.9 kilobase fragment than the 1.7 kilobase band secretes more of B subunit than several other clones as determined by bead ELIZA. This clone having the Accession No. B0011 (ATCC 202011) was subcultured in LB medium containing 10µg/ml of streptomycin sulfate for about 20 generations. The cells from this culture were plated on LB plate containing 10µg/ml of streptomycin sulfate and the colonies obtained were hybridized with the ctxB gene probe. All colonies hybridized indicating the stability of the integrated pGT27 after 20 generations. Genomic DNA from the recombinant *V*. *cholerae* clone having the accession no. B0011 was isolated and digested with XbaI, SalI and HpaI restriction enzymes separately. The Southern blot of the above digestions was hybridized with ctxB probe and hlyA probe in separate experiments.

Cholera vaccine having the Accession No. B0011 (ATCC 202011) was passaged (subcultured) in LB medium 56 times without any drug, each passage representing about 20 generations. About 1500 single colonies were checked for presence of ctxB by hybridization with the ctx Pr-B construct fragment as the probe. All of them hybridized with the probe indicating that ctx Pr-B construct was stably maintained even after about ≥1000 generations of growth in the absence of ampicillin.

V. *cholerae* having the Accession No. B0011 (ATCC 202011) was chosen as vaccine strain. It was tested by Rabbit ileal loop assay to determine whether the strain having the Accession No. B0011 (ATCC 202011) was reactogenic. Outbred New Zealand white rabbits of either sex weighing between 1.5 to 2.5 kg body weight were selected for rabbit ileal loop assays. Experimental animals were starved for 36 hours but were given water ad libitum. Surgery was done under anaesthesia given intravenously. The ileal loop procedure was essentially similar to that described by S.N. De [De, S.N., Nature, 183, 1533-1534 (1959)]. The intestine was brought out through a midline incision. The selected small intestinal portion were washed carefully with warm (37°C) sterile PBS (0.1 M, pH 7.4). A total of 8 loops were prepared in each rabbit. The length of the loop and the inter loop were 5 cms and 2 cms, respectively. One ml of live bacterial cell suspension containing about 10⁸ cells was then introduced in each loop. The known *V. cholerae* 569B was used as the positive control and sterile PBS (0.01 M, pH 7.4) was used as the negative control. After inoculation, the small intestine of the animal was introduced carefully inside the open abdomen and then the incision was sutured. Animals were kept in their cages and supplied with water. Animals were sacrified after 18 to 20 hours and an index of fluid accumulation (FA) was calculated from the ratio of loop fluid volume to loop length wichi was expressed as ml/cm. A test preparation was considered positive if the ratio was > 0.9. Results were discarded if control reactions were inappropriate. The vaccine strain having the Accession No. B0011 (ATCC 202011) was tested twice in the ileum of two different rabbits. The vaccine strain having the Accession No. B0011 (ATCC 202011) did not induce fluid accumulation. This attested the fact that the vaccine construct produces the B subunit but as is expected this subunit is inocuous and incapable of inducing fluid accumulation.

The next series of experiments were conducted to determine the colonization ability and protective ability of the vaccine using in vivo rabbit model. For this purpose outbred New Zealand white rabbits of either sex weighing between 1.7 to 2.5 kg were selected for the colonization experiments. All the animals were acclimatized in the laboratory for a week. The experimental rabbits were treated with a course of metronidazole (125 mg/rabbit/day) and sulfaquinoxaline sodium (464 mg/rabbit/day) and this course was repeated at an interval of two days to clean the animal of intestinal protozoan pathogens like Giardia and Coccidia. For preparing the oral inoculum the vaccine strain having the Accession No. B0011 (ATCC 202011) was grown overnight in tryptic soy broth (TSB, Difco, USA) at 37°C for 18 hours in an orbital shaker. Cells were harvested by centrifugation at 8000 rpm for 15 minutes. The pellet was suspended in sterile phosphate buffered saline (pH 7.4) and the bacterial density was estimated in a spectrophotometer at 540 nm and diluted using PBS to an optical density of approximately 10⁹ cells for use as the inoculum. Before oral immunization rabbits were fasted for 36 hours but water was given adlibitum; 35 minutes before oral inoculation each rabbit was anesthetized intramuscularly with ketamine (35 mg/kg body weight) and 4 mg/kg body weight of xylazine. After 5 minutes, the rabbits were administered 50 mg of cimetidine which is a H₂ receptor blocker and inhibits the secretion of HCl from peptic cells of the stomach. After 15 minutes a feeding tube (Accumark, Feeding Catheter, USA) was placed per os and 15 ml of a 5% solution of sodium bicarbonate (SRL India, sodium bicarbonate neutralizes HCl present in stomach) was introduced. At '0' time another 15 ml of 5% solution of sodium bicarbonate was given followed immediately by the bacterial inoculum suspended in 15 ml of 0.01 M PBS (pH-7.4). After 30 minutes, 2 ml of tincture of opium was given intraperitoneally. The rabbits were then returned to the cages and given limited amount of sterilized water and food. Both the experimental and control groups of rabbits were orally immunized with the vaccine strain having the Accesssion No. B0011 (ATCC 202011) on day 0, day 7 and day 14. The control group of rabbits were treated with uninoculated 15 ml of Tryptic soy broth (Difco, USA). On day 21 of the experiment, all the immunized animals were challenged by homologous or heterologous strains to determine the extent of protection. The ability of the animals to survive the challenge as well as the colonization ability of the organisms were studied.

To study the colonization of the test strains including the vaccine strain, the experimental rabbits were sacrificed after 18 hours of inoculation. The rabbits were anesthetized and the intestine was taken out after opening the abdomen surgically. Ten cm of the distal ileum was tied at both ends with umbilical tape (No. 11) and cut. The 10 centimeter ileum was placed into a beaker containing 10 ml of sterile 0.01 M PBS (pH 7.4). The pieces of intestine were opened longitudinally and washed gently. Serial dilutions were prepared from the washed materials. The tissue portion of ileum was weighed and homogenized with 10 ml of PBS (0.01 M, pH 7.4) and homogenized. Serial dilutions of the homogenate was prepared. The rabbits were finally killed using 2 ml of Euthanasia 6-solution 1 (Veterinary Lab./Inc. Kansas, USA) by intravenous injection. Neat and serial dilutions of the intestinal wash and homogenized material of ileum was plated on selective medium. The plates were incubated at 37°C and colony counts were made 24 hours later using a colony counter and expressed as colony forming units.

Homologous challenge studies were done with the same strain used for immunization and challenge.

Heterologous challenge studies were done with different strains used for immunization and challenge. Heterologous strains were chosen to represent both the biotypes (El Tor and classical) of *V. cholerae*.

Immunized rabbits challenged with homologous and heterologous strains were observed for clinical signs of diarrhoea. Diarrhoea was scored according to a grading system using the following characteristics. Stools were graded as follows : grade 1 represents normal stool without diarrhoea; grade 2 represents diarrhoea with soft mushy stools also termed as moderate diarrhoea and grade 3 represents diarrhoea with catarrhal and watery diarrhoea being termed as severe diarrhoea. It was clearly observed that the vaccine strain having the Accession No. B0011 (ATCC 202011) did not induce diarrhoea when fed orally to rabbits. Based on these results challenge studies with parent strains of *V. cholerae* of 01 serotype and of both biotypes E1 Tor and classical were conduced in several batches. The data can be summarised as follows :
1. Rabbit immunized with vaccine strain having the Accession No. B0011 (ATCC 202011) were significantly protected from a challenge with a classical biotype and an ElTor biotype strain. This was evident from the absence of diarrhoea in the immunized rabbits while the controls rabbits had profound idarrhoea.
2. The colonization ability of the challenge strains in the immunized rabbits were significantly lower as compared to the control rabbit.
3. The non-reactogenicity of the vaccine strain having the Accession No. B0011 (ATCC 202011) was again evident by its inability to provoke diarrhoea in the control rabbits.
4. The immunogenicity of the vaccine strain having the Accession No. B0011 (ATCC 202011). Since there was a significant rise in antibody titre against lipopolysaccharide, outer membrane protein, whole cell lysate and cholera toxin in immune sera as compared to the preimmune sera of rabbits orally immunized with the vaccine strain.

In summary then *V. cholerae* strain having Accession No. B0011 (ATCC 202011) is a candidate cholera vaccine strain which is devoid of all known virulence genes except ctxB, is non-reactogenic in animal model, elaborates the immunogenic "B" subunit of the cholera toxin and is capable of affording full protection against both biotypes of *V. cholerae,* El Tor and classical, in RITARD model. The cholera vaccine having the Accession No. B0011 (ATCC 2.02011) can be grown in Luria broth containing 50µg/ml of ampicillin at 37°C temperature and stored in the same medium with 20% (Vol/Vol) glycerol at -70°C.

The examples illustrated above should not be construed to limit the scope of the present invention.

Some preferred embodiments are as follow:
A*.* A process for the preparation of a cholera vaccine useful for preventing cholera, which comprises:
   a) isolating *V. Cholerae* from the stool of a patient suffering from Cholera by spreading the stool on a selector medium specific for *V. Cholerae*;
   b) separating the non-toxinogenic *V. Cholerae* strain from the population of the *V. Cholerae* strains isolated as in step (a), the strain having been deposited at Microbial Type Culture Collection (MTCC) at the Institute of Microbial Technology (IMT), Chandigarh, India; a constituent laboratory of the applicants and having the Accession no. MTCC B0010 which has also been deposited at American Type Culture Collection, Rockville, Maryland, USA and having Accession no. ATCC 202010; and
   c) incorporating immunogenic cholera toxin (ctx) B subunit gene into the chromosome of the strain having the Accession no. MTCC B0010 (ATCC 202010) by conventional method to produce the vaccine.
B. A process as defined above, wherein the medium used in step (a) is selected from thiosulfate-citrate bile salts sucrose agar (TBCS), tellurite taurocholate gelatin agar (TTGA), Vibrio agar, sucrose tellurite teepol medium and polymyxin mannose tellurite agar and the like.
C. A process for the preparation of a vaccine using the parent strain of *V. cholerae* having MTCC Accession no. B0010 (ATCC 202010) by a series of genetic manipulations leading to the constructions of ctx subunit gene flanked by hlyA gene sequences, said process comprises :
   a) creating a construct of the ctxB subunit gene with its own SD sequence and the promoter of the ctx-operon by first cloning the cholera toxin (ctx) operon from the chromosome of *V. cholerae* 569B strain (National Institute of Cholera and Enteric Diseases) and the deleting the ctx A subunit gene from the cloned operon by inverse PCR;
   b) cloning of the target locus hemolysinA gene (hlyA) for the purpose of targeted recombination;
   c) disrupting the hlyA gene sequence by the ctxB subunit gene construct as obtained at step (iii);
   d) cloning of the disrupted hlyA construct that has the ctxB subunit gene, into the suicide plasmid vector;
   e) mobilising the suicide vector bearing the disrupted hlyA construct into the parent strain [obtained at step (ii)] by conjunction, for targeted integration of the ctxB gene construct at the hlyA gene; and
   f) identifying the recombinant *V. cholerae* clones with the integrated ctxB gene by hybridisation and polymerase chain reaction.

## Claims

1. A method of producing a recombinant *Vibrio cholerae* strain, suitable for use as a vaccine, the method comprising selecting a naturally-occurring *Vibrio cholerae* strain which is devoid of the virulence genes *zot, ace, cep, orfU, ctxA* and *ctxB* from a population of *Vibrio cholerae* strains and incorporating a gene encoding the immunogenic cholera toxin B (*ctxB*) subunit into the genetic material of the selected strain by recombination, wherein the recombinant strain expresses the immunogenic *ctxB* gene, but no other said virulence genes.

2. A method of producing a recombinant *Vibrio cholerae* strain, suitable for use as a vaccine, according to claim 1, wherein the naturally-occurring *Vibrio cholerae* strain selected is the strain having the Accession Number ATCC 202010.

3. A method of producing a recombinant *Vibrio cholerae* strain, suitable for use as a vaccine, according to claim 1 or 2, wherein the recombinant strain does not comprise the *ctxA* gene.

4. A method of producing a recombinant *Vibrio cholerae* strain, suitable for use as a vaccine, according to any preceding claim, wherein the *ctxB* gene is inserted into the *hylA* gene.

5. A method of producing a recombinant *Vibrio cholerae* strain, suitable for use as a vaccine, according to claim 4, wherein the expression of the *hylA* gene is thereby disrupted in the recombinant strain.

6. A method of producing a recombinant *Vibrio cholerae* strain, suitable for use as a vaccine, according to any preceding claim, wherein the naturally-occurring *Vibrio cholerae* strain selected has not been subject to a genetic engineering process comprising gene deletion.

7. A method of producing a recombinant *Vibrio cholerae* strain, suitable for use as a vaccine, according to any preceding claim, wherein the naturally-occurring *Vibrio cholerae* strain selected comprises no virulence genes and the recombinant strain comprises no virulence genes other than *ctxB.*

8. A method of producing a recombinant *Vibrio cholerae* strain, suitable for use as a vaccine, according to any preceding claim, wherein the naturally-occurring *Vibrio cholerae* strain selected and the recombinant strain comprise the *tcpA* gene.

9. A method of producing a recombinant *Vibrio cholerae* strain, suitable for use as a vaccine, according to any preceding claim, wherein the naturally-occurring *Vibrio cholerae* strain selected and the recombinant strain comprise the *toxR* gene.

10. A method of producing a recombinant *Vibrio cholerae* strain, suitable for use as a vaccine, according to any preceding claim, wherein the recombinant strain is non-reactogenic and is non-secretogenic, but is able to colonise the intestine.

11. A method of producing a recombinant *Vibrio cholerae* strain, suitable for use as a vaccine, according to any preceding claim, wherein the recombinant strain is an E1 Tor strain of *Vibrio cholerae.*

12. A recombinant *Vibrio cholerae* strain expressing the immunogenic *ctxB* gene and devoid of the virulence genes *zot, ace, cep, orfU* and *ctxA,* produced according to the method of any of claims 7-9.

13. A *Vibrio cholerae* vaccine comprising the recombinant *Vibrio cholerae* strain according to claim 12.

## Patentansprüche

1. Verfahren zum Erzeugen eines rekombinanten *Vibrio-cholerae-*Stammes, geeignet zur Verwendung als Impfstoff, wobei das Verfahren das Auswählen eines natürlich vorkommenden *Vibrio-cholerae-Stammes,* der frei von den Virulenzgenen *zot, ace, cep, orfU, ctxA* und *ctxB* ist, aus einer Population von *Vibrio-cholerae-*Stämmen und Einbringen eines Gens, codierend die immunogene Choleratoxin-B-(*ctxB*)-Untereinheit, in das genetische Material des ausgewählten Stammes durch Rekombination umfaßt, wobei der rekombinante Stamm das immunogene *ctxB*-Gen, aber keine anderen Virulenzgene exprimiert.

2. Verfahren zum Erzeugen eines rekombinanten *Vibrio-cholerae-Stammes,* geeignet zur Verwendung als Impfstoff, gemäß Anspruch 1, wobei der ausgewählte natürlich vorkommende *Vibrio-cholerae-Stamm* der Stamm mit der Zugangsnummer ATCC 202010 ist.

3. Verfahren zum Erzeugen eines rekombinanten *Vibrio-cholerae-*Stammes, geeignet zur Verwendung als Impfstoff, gemäß Anspruch 1 oder 2, wobei der rekombinante Stamm nicht das ctxA-Gen umfaßt.

4. Verfahren zum Erzeugen eines rekombinanten *Vibrio-cholerae-Stammes,* geeignet zur Verwendung als Impfstoff, gemäß einem der vorhergehenden Ansprüche, wobei das ctxB-Gen in das *hylA-*Gen insertiert wird.

5. Verfahren zum Erzeugen eines rekombinanten *Vibrio-cholerae-Stammes,* geeignet zur Verwendung als Impfstoff, gemäß Anspruch 4, wobei die Expression des *hylA-*Gens **dadurch** in dem rekombinanten Stamm unterbrochen wird.

6. Verfahren zum Erzeugen eines rekombinanten *Vibrio-cholerae-Stammes,* geeignet zur Verwendung als Impfstoff, gemäß einem der vorhergehenden Ansprüche, wobei der ausgewählte natürlich vorkommende *Vibrio-cholerae-Stamm* nicht Gegenstand eines Gendeletion umfassenden gentechnischen Verfahrens gewesen ist.

7. Verfahren zum Erzeugen eines rekombinanten *Vibrio-cholerae-Stammes,* geeignet zur Verwendung als Impfstoff, gemäß einem der vorhergehenden Ansprüche, wobei der ausgewählte natürlich vorkommende *Vibrio-cholerae-Stamm* keine Virulenzgene umfaßt und der rekombinante Stamm keine Virulenzgene anders als *ctxB* umfaßt.

8. Verfahren zum Erzeugen eines rekombinanten *Vibrio-cholerae-Stammes,* geeignet zur Verwendung als Impfstoff, gemäß einem der vorhergehenden Ansprüche, wobei der ausgewählte natürlich vorkommende *Vibrio-cholerae-Stamm* und der rekombinante Stamm das *tcpA-*Gen umfassen.

9. Verfahren zum Erzeugen eines rekombinanten *Vibrio-cholerae-Stammes,* geeignet zur Verwendung als Impfstoff, gemäß einem der vorhergehenden Ansprüche, wobei der ausgewählte natürlich vorkommende *Vibrio-cholerae-Stamm* und der rekombinante Stamm das *toxR*-Gen umfassen.

10. Verfahren zum Erzeugen eines rekombinanten *Vibrio-cholerae-Stammes,* geeignet zur Verwendung als Impfstoff, gemäß einem der vorhergehenden Ansprüche, wobei der rekombinante Stamm nicht reaktogen ist und nicht sekretogen ist, aber imstande ist, den Darm zu besiedeln.

11. Verfahren zum Erzeugen eines rekombinanten *Vibrio-cholerae-Stammes,* geeignet zur Verwendung als Impfstoff, gemäß einem der vorhergehenden Ansprüche, wobei der rekombinante Stamm ein El-Tor-Stamm von *Vibrio cholerae* ist.

12. Rekombinanter *Vibrio-cholerae-*Stamm, der das immunogene ctxB-Gen ausdrückt und frei von den Virulenzgenen *zot, ace, cep, orfU* und *ctxA* ist, erzeugt gemäß dem Verfahren nach einem der Ansprüche 7-9.

13. *Vibrio-cholerae-*Impfstoff*,* umfassend den rekombinanten *Vibrio-cholerae-*Stamm gemäß Anspruch 12.

## Revendications

1. Procédé de production d'une souche recombinante de *Vibrio cholerae,* convenant à être utilisée en tant que vaccin, le procédé comprenant sélectionner une souche de *Vibrio cholerae* survenant naturellement qui est dépourvue des gènes de virulence *zot, ace, cep, orfU, ctxA* et *ctxB,* parmi une population de souches de *Vibrio cholerae* et incorporer un gène codant la sous-unité B immunogène de la toxine du choléra (*ctxB*) dans le matériel génétique de la souche sélectionnée par recombinaison, où la souche recombinante exprime le gène *ctxB* immunogène mais aucun des autres dits gènes de virulence.

2. Procédé de production d'une souche recombinante de *Vibrio cholerae,* convenant à être utilisée en tant que vaccin, selon la revendication 1, où la souche de *Vibrio cholerae* survenant naturellement sélectionnée est la souche ayant le numéro d'accès ATCC 202010.

3. Procédé de production d'une souche recombinante de *Vibrio cholerae,* convenant être utilisée en tant que vaccin, selon la revendication 1 ou 2, où la souche recombinante ne comprend pas le gène *ctxA.*

4. Procédé de production d'une souche recombinante de *Vibrio cholerae,* convenant être utilisée en tant que vaccin, selon l'une quelconque ces revendications précédentes, où le gène *ctxB* est introduit dans le gène *hylA.*

5. Procédé de production d'une souche recombinante de *Vibrio cholerae,* convenant être utilisée en tant que vaccin, selon la revendication 4, où l'expression du gène *hylA* est ainsi rompue dans la souche recombinante.

6. Procédé de production d'une souche recombinante de *Vibrio cholerae,* convenant être utilisée en tant que vaccin, selon l'une quelconque des revendications précédentes, où la souche de *Vibrio cholerae* survenant naturellement sélectionnée n'a pas été soumise à un procédé de génie génétique comprenant une délétion génique.

7. Procédé de production d'une souche recombinante de *Vibrio cholerae,* convenant être utilisée en tant que vaccin, selon l'une quelconque des revendications précédentes, où la souche de *Vibrio cholerae* survenant naturellement sélectionnée ne comprend aucun gène de virulence et la souche recombinante ne comprend aucun gène de virulence autre que *ctxB.*

8. Procédé de production d'une souche recombinante de *Vibrio cholerae,* convenant être utilisée en tant que vaccin, selon l'une quelconque des revendications précédentes, où la souche de *Vibrio cholerae* survenant naturellement sélectionnée et la souche recombinante comprennent le gène *tcpA.*

9. Procédé de production d'une souche recombinante de *Vibrio cholerae,* convenant être utilisée en tant que vaccin, selon l'une quelconque des revendications précédentes, où la souche de *Vibrio cholerae* survenant naturellement sélectionnée et la souche recombinante comprennent le gène *toxR.*

10. Procédé de production d'une souche recombinante de *Vibrio cholerae,* convenant être utilisée en tant que vaccin, selon l'une quelconque des revendications précédentes, où la souche recombinante est non réactogène et non sécrétogène mais est capable de coloniser l'intestin.

11. Procédé de production d'une souche recombinante de *Vibrio cholerae,* convenant être utilisée en tant que vaccin, selon l'une quelconque des revendications précédentes, où la souche recombinante est une souche El Tor de *Vibrio cholerae.*

12. Souche recombinante de *Vibrio cholerae* exprimant le gène immunogène *ctxB* et dépourvue des gènes de virulence *zot, ace, cep, orfU* et *ctxA,* produite selon le procédé de l'une quelconque des revendications 7-9.

13. Vaccin de *Vibrio cholerae* comprenant la souche recombinante de *Vibrio cholerae* selon la revendication 12.
